**Europäisches Patentamt**

**European Patent Office**

**Office européen des brevets**

(19)

(11) Veröffentlichungsnummer: **0 108 318**
**B1**

# EUROPÄISCHE PATENTSCHRIFT

(12)

(45) Veröffentlichungstag der Patentschrift:
08.01.86

(51) Int. Cl.⁴: **A 61 K 7/22,** A 61 K 7/26

(21) Anmeldenummer: 83110557.2

(22) Anmeldetag: 22.10.83

(54) **Zahn- und Mundpflegemittel.**

(30) Priorität: **06.11.82 DE 3241017**

(43) Veröffentlichungstag der Anmeldung:
**16.05.84 Patentblatt 84/20**

(45) Bekanntmachung des Hinweises auf die Patenterteilung:
**08.01.86 Patentblatt 86/2**

(84) Benannte Vertragsstaaten:
**AT BE CH DE FR GB IT LI LU NL SE**

(56) Entgegenhaltungen:
**DE - A - 2 409 755**
**DE - B - 1 139 609**

**CHEMICAL ABSTRACTS, Band 94, Nr. 18, Mai 1981,
Seite 360, Nr. 145174k, Columbus, Ohio, USA, G.
PROSERPIO et al.: "Cosmetic uses of horse-chestnut
(Aesculus hippocastanum) extracts, of escin and of the
cholesterol/escin complex"**

(73) Patentinhaber: **Blendax-Werke R. Schneider GmbH &
Co., Rheinallee 88, D-6500 Mainz (DE)**

(72) Erfinder: **Weinert, Wolfgang, Dr., Königsberger
Strasse 22, D-6208 Bad Schwalbach (DE)**

## Beschreibung

Die vorliegende Erfindung betrifft ein neues Zahn- und Mundpflegemittel, das auf das Zahnfleisch eine gesunderhaltende Wirkung ausübt und insbesondere die Ursachen für Zahnfleischerkrankungen beseitigt und Zahnfleischbluten verhindert bzw. vermindert.

Mund- und Zahnpflegemittel mit derartiger Zielsetzung sind bereits seit langem bekannt und auch im Handel.

Für diese Zahnpasten wurden verschiedene Wirkstoffe vorgeschlagen. Ein in diesem Zusammenhang anerkannter Stoff, der auch Eingang in die Praxis gefunden hat, ist Harnstoff. Von dieser Substanz ist nicht nur beschrieben, dass sie eine kariesprophylaktische und zahnsteinverhütende Wirkung aufweist (vgl. «Journal of Periodontology», Vol. 37 (1966, S. 20-33)), sondern sie besitzt auch eine keratinisierende Wirkung, d.h., beim Einsatz in Zahn- und Mundpflegemitteln in genügender Konzentration wirkt sie auf das Zahnfleisch festigend, was sich durch eine Erhöhung des Keratinisations-Indexes signifikant nachweisen lässt.

Es wurde nun gefunden, dass sich die Wirkung von Harnstoff enthaltenden Zahn- und Mundpflegemitteln auf das Zahnfleisch, insbesondere im Sinne einer Verhinderung von Zahnfleischbluten, noch verbessern lässt, wenn man diesen Mitteln Rosskastanienextrakt, vorzugsweise in einer Menge zwischen etwa 0,1 und 1 Gew.-%, bezogen auf die Gesamtzusammensetzung, zusetzt.

Die Verwendung von Rosskastanienextrakt in Zahn- und Mundpflegemitteln ist an sich ebenfalls bereits bekannt und beispielsweise in der DE-AS Nr. 1139609 beschrieben.

Rosskastanienextrakt enthaltende Zahnpasten der dort beschriebenen Art haben jedoch keine praktische Anwendung gefunden.

Es war daher höchst überraschend und nicht vorhersehbar, dass durch die Kombination der beiden Wirkstoffe Harnstoff und Rosskastanienextrakt in einem Zahn- und Mundpflegemittel, insbesondere einer Zahnpasta, eine derartige synergistische Wirkungssteigerung im Hinblick auf die Zahnfleischbehandlung erreicht werden konnte.

Eine besonders günstige Wirkung wurde erreicht, wenn eine Zahnpasta mit einem Gehalt von mindestens 5 Gew.-% Harnstoff, vorzugsweise 8 Gew.-% Rosskastanienextrakt, bezogen auf die Gesamtzusammensetzung, einer klinischen Untersuchung unterzogen wurde.

Unter Rosskastanienextrakt werden im Rahmen der vorliegenden Erfindung die alkoholischen bzw. wässerig-alkoholischen Extrakte der geschälten Samen von Aesculus Hippocastanum verstanden.

Es ist vorteilhaft, aus diesen alkoholischen bzw. wässerig-alkoholischen Extrakten die enthaltenen Gerbstoffe und Eiweisse abzutrennen, jedoch ist dies zur Erreichung der erfindungsgemässen Wirkung nicht unbedingt erforderlich.

Bei dem eingesetzten Rosskastanienextrakt handelt es sich vorzugsweise um einen Trockenextrakt, der auf einen Aescingehalt zwischen etwa 17 und 19% standardisiert ist, jedoch lässt sich ohne weiteres auch ein Flüssigextrakt verwenden, wobei die bevorzugte Menge dann sinngemäss auf die standardisierte Menge des Trockenextraktes umgerechnet werden muss.

Eine Beschreibung des Rosskastanienextraktes und seiner pharmakologischen Eigenschaften findet sich in «Hagers Handbuch der pharmazeutischen Praxis», 4. Neuausgabe, II. Band (Springer-Verlag, 1969), S. 1110 bis 1115.

Wie bereits ausgeführt, liegt die bevorzugte Menge an Rosskastanienextrakt in den erfindungsgemässen Zahn- und Mundpflegemitteln bei etwa 0,1 bis 1,0 Gew.-% der Gesamtzusammensetzung. Werden höhere Anteile als 1% verwendet, besteht die Gefahr, dass Schleimhautreizungen auftreten; eine optimale Dosierung liegt zwischen etwa 0,5 und 0,6 Gew.-%, beispielsweise bei 0,55 Gew.-% der Gesamtzusammensetzung.

Der Anteil an Harnstoff in dem erfindungsgemässen Zahn- und Mundpflegemittel liegt bei mindestens 5 Gew.-% der Gesamtzusammensetzung; eine besonders günstige Wirkung hat sich bei der Kombination von 8% Harnstoff mit 0,55 Gew.-% Rosskastanienextrakt herausgestellt. Im allgemeinen wird der Gehalt an Harnstoff 15 Gew.-%, vorzugsweise 10 Gew.-%, bezogen auf die Gesamtzusammensetzung, nicht überschreiten.

Obwohl prinzipiell jede geeignete Applikationsform eines Zahn- und Mundpflegemittels mit einem Gehalt an Harnstoff und Rosskastanienextrakt verwendet werden kann, wie beispielsweise Mundwasser, Mundspray oder Zahnpulver, wird der Applikationsform Zahnpasta der Vorzug gegeben.

Eine solche Zahnpasta kann opak oder eine durch Verwendung geeigneter, in ihren Brechungsindizes mit dem Brechungsindex des Trägermaterials übereinstimmender Poliermittel hergestellte transparente Zahnpasta sein.

Eine besonders geeignete Zahnpasta ist in der LU-PS Nr. 82933 beschrieben; diese enthält das Poliermittel Calciumcarbonat sowie mindestens 5 Gew.-% Harnstoff, etwa 0,5 bis 1,6 Gew.-% eines Alkalisalzes einer höheren Fettsäure mit etwa 12 bis etwa 18 Kohlenstoffatomen, ist im wesentlichen frei von synthetischen Tensiden und weist einen pH-Wert im alkalischen Bereich von mindestens 7,5, vorzugsweise zwischen 7,5 und 9,5, auf.

Es ist jedoch auch möglich, Zahnpasten auf anderer Pastengrundlage einzusetzen, die als Poliermittel beispielsweise Alkalialuminiumsilikate wie solche vom Zeolith-Typ A, beschrieben in den EP-PSen Nrn. 2690 und 3023, verschiedene Calciumphosphate wie Dicalciumorthophosphat in Form seines Dihydrats oder wasserfrei, Tricalciumphosphat, Calciumpyrophosphat, unlösliche Alkalimetaphosphate, Aluminiumoxid oder Aluminiumoxidtrihydrat, Siliciumdioxide verschiedener Modifikationen wie Siliciumdioxid-Xerogele, -Hydrogele oder gefällte Siliciumdioxide, oder pulverförmige Kunststoffe enthalten.

Es können selbstverständlich auch Poliermittelgemische aus den genannten Substanzen eingesetzt werden, beispielsweise ein Gemisch aus Calciumcarbonat und synthetischem Zeolith A im Verhältnis von etwa 1:1.

Der Poliermittelanteil in den erfindungsgemässen Zahnpasten liegt vorzugsweise zwischen etwa 20 und 60 Gew.-% der Gesamtzusammensetzung.

Wie bereits angedeutet, stellt es eine bevorzugte Ausführungsform der vorliegenden Erfindung dar, die synergistische Kombination aus Harnstoff und Rosskastanienextrakt solchen Zahnpasten einzuverleiben, die nur einen geringen oder keinen Anteil an synthetischen oberflächenaktiven Substanzen enthalten, sondern gegebenenfalls Alkalisalze höherer Fettsäuren, beispielsweise solche · von Laurinsäure, Myristinsäure, Palmitinsäure, Stearinsäure oder Gemischen derselben, beispielsweise Cocosölfettsäuren oder Talgfettsäuren, enthalten. Derartige Salze höherer Fettsäuren sind vorzugsweise in einer Menge zwischen etwa 0,5 und 1,5 Gew.-% der Gesamtzusammensetzung vorhanden.

Es ist jedoch selbstverständlich auch möglich, die üblichen in Zahnpasten eingesetzten oberflächenaktiven Verbindungen in Mengen bis zu etwa 2 Gew.-% der Gesamtzusammensetzung zu verwenden, gegebenenfalls im Gemisch mit den genannten Fettsäuresalzen. Solche synthetischen oberflächenaktiven Stoffe sind beispielsweise Alkylsulfate, Alkylethersulfate, Olefinsulfonate, Natriumlaurylsarcosinat oder ampholytische, nichtionische oder kationaktive Verbindungen.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M.S. Balsam und E. Sagarin «Cosmetics - Science and Technology», 2nd Ed., Vol. 1, S. 423 bis 533 (1972), auf das hier ausdrücklich Bezug genommen wird.

Gleiches gilt hinsichtlich der in Zahnpasten üblicherweise in Mengen zwischen etwa 10 und etwa 35 Gew.-% zum Einsatz gelangenden Feuchthaltemittel, wie Glycerin, Diole wie 1,4-Butandiol oder 1,2-Propandiol oder Zuckeralkohole wie Sorbit, Mannit oder Xylit und Polyglykole mit niederen Molekulargewichten, ebenso für Verdickungsmittel, deren Mengenanteil in Zahnpasten zwischen etwa 0,25 und etwa 5 Gew.-% der Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Alkalisalze der Polyacrylsäure sowie anorganische Verdickungsmittel, beispielsweise kolloidales Magnesiumaluminiumsilikat oder disperses Siliciumdioxid.

In den erfindungsgemässen Zahn- und Mundpflegemitteln können selbstverständlich auch weitere Wirkstoffe Verwendung finden. Solche sind insbesondere die bekannten kariesprophylaktischen Fluoride, vorzugsweise in einer solchen Menge, dass die Konzentration an reinem Fluor im Mittel etwa 0,05 bis etwa 1 Gew.-%, vorzugsweise 0,1 bis 0,5 Gew.-%, des Mittels beträgt.

Geeignete Fluorverbindungen sind insbesondere die verschiedenen Salze der Monofluorphosphorsäure, insbesondere Natrium-, Kalium-, Lithium, Calcium- und Aluminiummono- und -difluorphosphat sowie die verschiedenen, Fluor in ionisch gebundener Form enthaltenden Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsprodukte dieser Fluoride untereinander und mit anderen Fluorverbindungen, beispielsweise Alkalimanganfluoride.

Auch organische Fluorverbindungen können mit Erfolg eingesetzt werden, insbesondere die bekannten Additionsprodukte aus langkettigen Aminen oder Aminosäuren und Fluorwasserstoff, Monoethanolamindihydrofluorid oder Monoethyltriethylammoniumfluorid.

Weitere, in den erfindungsgemässen Zahn- und Mundpflegemitteln einsetzbare Stoffe sind Zahnbelag entfernende Substanzen, beispielsweise die unter den Trivialnamen «Chlorhexidin» oder «Alexidin» bekannten Bisguanide, 1,6-Di-4'-(chlorphenyldiguanido)hexan oder 1,6-Di-(2-ethylhexylidiguanido)hexan bzw. deren vorzugsweise wasserlösliche Salze, Mittel zur Verhinderung von Zahnsteinbildung wie Hydroxyethan-1,1-diphosphonsäure oder Alkylentetramethylenphosphonsäuren und deren wasserlösliche Salze, Allantoin, und Azulen.

Im folgenden werden einige Beispiele gegeben, die das Wesen der vorliegenden Anmeldung und ihre vorteilhafte Wirkung charakterisieren:

*Beispiel 1:*

*Opake Zahnpasta*

| | (Gew.-%) |
|---|---|
| Methylhydroxyethylcellulose | 1,00 |
| Calciumcarbonat | 42,00 |
| Harnstoff | 8,00 |
| Allantoin | 0,30 |
| Natriumlaurat | 0,65 |
| Natriumbenzoat | 0,30 |
| Methyl-p-hydroxybenzoat | 0,15 |
| Saccharin-Natrium | 0,05 |
| Sorbit, 70%ig | 8,00 |
| Rosskastanienextrakt (Standardisiert auf 18% Aescin) | 0,60 |
| Kolloidales Siliciumdioxid | 0,35 |
| Aromastoffe | 1,00 |
| Wasser | 37,60 |

*Beispiel 2:*

*Opake Zahnpasta*

| | (Gew.-%) |
|---|---|
| Harnstoff | 6,00 |
| Natriummonofluorphosphat | 0,75 |

|  | (Gew.-%) |
|---|---|
| Allantoin | 0,10 |
| Natriumstearat/-laurat (1:1) | 0,70 |
| Natriumbenzoat | 0,25 |
| Calciumcarbonat | 40,00 |
| Methyl-p-hydroxybenzoat | 0,15 |
| Natriumcyclamat | 0,10 |
| Calciumsilikat | 0,50 |
| Hydroxyethylcellulose | 1,10 |
| Sorbit, 70%ig | 9,00 |
| Aromagemisch | 1,00 |
| Rosskastanienextrakt | 0,55 |
| Wasser | 39,80 |

**Beispiel 3:**

*Opake Zahnpasta*

|  | (Gew.-%) |
|---|---|
| Calciumcarbonat | 22,50 |
| Synthetischer Zeolith A (entsprechend EP-PS 3023; $Na_{12}(A10_2)_{12} (SiO)_{12} \cdot 27H_2O$) | 16,50 |
| Sorbit, 70%ig | 7,50 |
| Glycerin | 3,50 |
| Carboxymethylcellulose | 1,20 |
| Benzoesäure | 0,30 |
| Methyl-p-hydroxybenzoat | 0,10 |
| Saccharin-Natrium | 0,05 |
| Natriummonofluorphosphat | 1,25 |
| Kolloidale Kieselsäure | 0,20 |
| Harnstoff | 10,00 |
| Rosskastanienextrakt | 0,80 |
| Aromastoffe | 1,00 |
| Natriumlaurylsulfat, 86%ig | 1,20 |
| Wasser | 33,90 |

**Beispiel 4:**

*Transparente Zahnpasta*

|  | (Gew.-%) |
|---|---|
| Carboxymethylcellulose | 0,50 |
| Natriumbenzoat | 0,15 |
| Polyethylenglykol 400 | 5,00 |
| Glycerin | 45,00 |
| Harnstoff | 7,50 |
| Rosskastanienextrakt | 0,55 |
| Allantoin | 0,15 |
| Guajazulen | 0,05 |
| Saccharin-Natrium | 0,07 |
| Natriumlauroylsarkosinat | 1,10 |
| Phenylsalicylat | 0,10 |

|  | (Gew.-%) |
|---|---|
| Aromagemisch | 1,00 |
| 10%ige blaue Farbstoff-Lösung | 0,03 |
| Dehydratisiertes Siliciumdioxid-Gel (Oberfläche 290 m²/g; Mittl. Teilchendurchmesser 6 µ) | 20,00 |
| Wasser | 18,80 |

**Beispiel 5:**

*Opake Zahnpasta*

|  | (Gew.-%) |
|---|---|
| Chlorhexidindigluconat | 0,10 |
| Harnstoff | 8,00 |
| Rosskastanienextrakt | 0,65 |
| Allantoin | 0,20 |
| Natriumfluorid | 0,30 |
| Aluminiumoxidtrihydrat | 25,00 |
| Zeolith A (nach EP-PS 3023; $Na_{12}(A1O_2)_{12} (SiO_2)_{12} \cdot 27H_2O$) | 15,00 |
| Medizinische Seife (DAB 6) | 0,70 |
| Natriumsulforicinoleat | 0,30 |
| Hydroxyethylcellulose | 1,05 |
| N-Prophyl-p-hydroxybenzoat | 0,15 |
| Methyl-p-hydroxybenzoat | 0,15 |
| Natriumbenzoat | 0,15 |
| Saccharin-Natrium | 0,05 |
| Glycerin | 8,50 |
| Sorbit, 70%ig | 7,50 |
| Aromazusammensetzung | 1,20 |
| Wasser | 31,00 |

*Klinische Untersuchung zum Nachweis des synergistischen Effekts der erfindungsgemässen Kombination:*

60 erwachsene Versuchspersonen wurden in 3 Gruppen zu je 20 Probanden eingeteilt.

Bei jedem Probanden wurden zu Versuchsbeginn der Papillenblutungsindex und die Sulcus-Fluid-Flow-Rate bestimmt, die beide aussagefähige Indizes für den Gesundheitszustand des Zahnfleisches darstellen.

Dann erhielt jede der Gruppen mit den Decknamen A, B und C bezeichnete Zahnpasten zur zweimaligen täglichen Anwendung, wobei an die Probanden einheitliche Zahnbürsten ausgegeben wurden.

Nach 30 Tagen wurden die Versuchspersonen erneut untersucht und die obengenannten Indizes ermittelt. Dabei ergab sich folgendes Resultat:

| Tag | Paste A | | Paste B | | Paste C | |
|---|---|---|---|---|---|---|
|  | 0 | 30 | 0 | 30 | 0 | 30 |
| Sulcus-Fluid-Flow-Rate | 3,20±0,22 | 3,34±0,23 | 3,22±0,23 | 2,02±0,56 | 3,25±1,07 | 3,07±0,27 |
| Papillenblutungsindex | 1,88±0,33 | 2,44±0,65 | 1,90±0,62 | 1,35±0,36 | 1,89±0,63 | 1,86±0,60 |

Paste B war eine Zahnpasta nach Beispiel 1; Paste A war eine Zahnpasta nach Beispiel 1, jedoch ohne Harnstoff, wobei der Gewichtsausgleich durch Erhöhung des Wassergehaltes erfolgte; Paste C war eine Zahnpasta nach Beispiel 1, jedoch ohne Rosskastanienextrakt, wobei der Gewichtsausgleich ebenfalls durch Erhöhung des Wassergehaltes erfolgte.

Wie aus der Tabelle eindeutig hervorgeht, wird durch die erfindungsgemässe Zahnpasta nach Beispiel 1 eine signifikante, überraschende Herabsetzung der relevanten Indizes bezüglich der Blutungsneigung des Zahnfleisches gegenüber den dem Stand der Technik entsprechenden, lediglich die Einzelkomponenten des erfindungsgemässen synergistischen Gemisches enthaltenden Zahnpasten erzielt.

## Patentansprüche

1. Zahn- und Mundpflegemittel auf Basis der üblichen Zusatz- und Aufbaustoffe, enthaltend mindestens 5 Gew.-%, bezogen auf die Gesamtzusammensetzung, an Harnstoff, gekennzeichnet durch einen zusätzlichen Gehalt an Rosskastanienextrakt.

2. Zahn- und Mundpflegemittel nach Anspruch 1, gekennzeichnet durch einen zusätzlichen Gehalt an 0,1 bis 1 Gew.-%, bezogen auf die Gesamtzusammensetzung, an Rosskastanienextrakt.

3. Zahn- und Mundpflegemittel nach Anspruch 2, gekennzeichnet durch einen Gehalt an 0,5 bis 0,6 Gew.-%, bezogen auf die Gesamtzusammensetzung, an Rosskastanienextrakt.

4. Zahn- und Mundpflegemittel nach einem oder mehreren der vorangegangenen Ansprüche, vorliegend in Form einer Zahnpasta auf wässeriger Basis, enthaltend Calciumcarbonat als alleiniges oder überwiegendes Poliermittel, 5 bis 15 Gew.-% Harnstoff, 0,5 bis 1 Gew.-%, bezogen auf die Gesamtzusammensetzung eines Alkalisalzes einer $C_{12}$- bis $C_{22}$-Fettsäure, mit einem pH-Wert oberhalb 7,5, gekennzeichnet durch einen zusätzlichen Gehalt an Rosskastanienextrakt.

## Claims

1. Tooth and mouth care agent on the basis of the usual additives and materials containing at least 5% by weight, calculated to the total composition, of urea, characterized by an additional content of horse chestnut extract.

2. Tooth and mouth care agent according to claim 1, characterized by an additional content of 0.1 to 1.0% by weight, calculated to the total composition, of horse chestnut extract.

3. Tooth and mouth care agent according to claim 2, characterized by an additional content of 0.5 to 0.6% by weight, calculated to the total composition, of horse chestnut extract.

4. Tooth and mouth care agent according to one or more of the preceding claims in the form of a toothpaste on an aqueous basis, containing calcium carbonate as sole or major polishing agent, 5 to 15% by weight of urea, 0.5 to 1% by weight, calculated to the total composition, of an alkali salt of a $C_{12}$- to $C_{22}$-fatty acid with a pH-value above 7.5, characterized by an additional content of horse chestnut extract.

## Revendications

1. Agent pour l'hygiène dentaire et buccale sur la base des additifs et des matières premières usuelles qui contient au minimum 5% en poids d'urée, caractérisé par une teneur supplémentaire d'extrait du marron d'Inde.

2. Agent pour l'hygiène dentaire et buccale selon la revendication 1, caractérisé par une teneur supplémentaire de 0,1-1% en poids d'extrait du marron d'Inde relatif à la composition totale.

3. Agent pour l'hygiène dentaire et buccale selon la revendication 2, caractérisé par une teneur de 0,5-0,6% en poids d'extrait du marron d'Inde relatif à la composition totale.

4. Agent pour l'hygiène dentaire et buccale selon une ou plusieurs des revendications susmentionnées présent sous forme d'une pâte dentifrice sur base aqueuse qui contient du carbonate de calcium comme agent polissant unique ou prépondérant, 5-15% en poids d'urée, 0,5-1% en poids relatif à la composition totale d'un sel alcalin d'un C12-C22 acide gras avec une valeur pH dépassant 7,5, caractérisé par une teneur supplémentaire d'extrait du marron d'Inde.